# EUROPEAN PATENT SPECIFICATION

(11) **EP 1 152 056 B1**
(45) Date of publication and mention of the grant of the patent: **24.03.2010**
(21) Application number: 00981979.8
(22) Date of filing: 07.12.2000
(51) Int. Cl.: C12N 15/00

(54) **METHOD FOR MODIFYING GENETIC CHARACTERISTICS OF AN ORGANISM**
VERFAHREN ZUR VERANDERUNG DER GENETISCHEN CHARAKTERISTIKA EINES ORGANISMUS
PROCEDE DE MODIFICATION DES PROPRIETES GENETIQUES D'UN ORGANISME

(30) Priority: 09.12.1999 RU 99125827
(43) Date of publication of application: 07.11.2001
(73) Proprietor: UCHREZHDENIE ROSSIISKOI AKADEMII NAUK INSTITUT MOLEKULYARNOI BIOLOGII IM. V.A. ENGELGARDTA RAN (IMB RAN), Moscow 117984 (RU)
(72) Inventor: CHURIKOV, Nikolai Andreevich, Moscow, 117418 (RU)
(74) Representative: Dahnér, Christer
(86) International application number: PCT/RU2000/000504
(87) International publication number: WO 2001/042443

(56) References cited:
- WO-A1-98/53083
- RU-A1- 94 046 161
- TCHURIKOV N A ET AL: "THE FORMATION OF PARALLEL RNA-RNA DUPLEXES IN VITRO" JOURNAL OF BIOMOLECULAR STRUCTURE & DYNAMICS, ADENINE PRESS, NEW YORK, NY, US, vol. 13, no. 3, December 1995 (1995-12), pages 507-513, XP001055465 ISSN: 0739-1102
- TCHURIKOV NA ET AL: "Gene-specific silencing by expression of parallel complementary RNA in Escherichia coli." THE JOURNAL OF BIOLOGICAL CHEMISTRY, vol. 275, no. 34, 25 August 2000 (2000-08-25), pages 26523-26529, XP002190363
- CHURIKOV NA ET AL: "Generation of Kruppel phenocopies by injecting into Drosophila embryos RNA complementary to mRNA in parallel orientation." GENETICA, vol. 36, no. 2, February 2000 (2000-02), pages 149-154, XP002190364 -& CHURIKOV NA ET AL: "GENERATION OF KRUPPEL PHENOCOPIES BY INJECTING INTO DROSOPHILA EMBRYOS RNA COMPLEMENTARY TO mRNA IN PARALEL ORIENTATION" GENETIKA, vol. 36, no. 2, - February 2000 (2000-02) pages 149-154, XP001062956
- PONOMORENKO N.A. ET AL.: "The formation of a parallel RNA-RNA duplex in vivo in Escherichia coli cells" DOKLADI AKADEMII NAUK, vol. 327, no. 4-6, 1 January 1992 (1992-01-01), - 1 January 1992 (1992-01-01) pages 584-588,

## Description

### Field of invention

The invention concerns with the molecular biology, molecular genetics and biotechnology and can be used in the gene-therapy in the medicine and the agriculture or in the industrial biotechnology for a gene-specific silencing of the disease-related genes or the genes interfering a buildup of a product, respectively.

### Background of invention

Different approaches for changing the genetic properties of an organism are used. Some of them assume the damage of a gene. The example of the latter is so-called genes' knockout. The approach utilizes the damage (mutation) of a selected gene in germ-line or stem cells and thus cannot be used in the most cases for the developed organism [L.V. Varga, S. Toth, I. Novak, A. Falus, Immunol. Lett., 1999, vol. 69, p. 217; J. Osada, N. Maeda, Methods Mol. Biol., 1998, vol.110, p. 79].

During recent years an increased attention is attracted by another approach for changing the genetic properties of an organism by RNA interference, leading to a gene-specific silencing by changes of the regulation of an undamaged gene [M.K. Montgomery, A. Fire, Trends in Genetics, 1998, vol.14, p. 255; P. Sharp, Genes & Development, 1999, vol.13, p.139]. RNA interference can be used for gene-specific silencing at any stage of development, including the adults. The increased attention to RNA interference is due to the fact that these studies serendipitously uncovered the ancient mechanisms of gene regulation. The physiological role of this mechanism of regulation could include the local changes of chromosomal structures, transcription activity, RNA processing and transport into the cytoplasm and RNA stability.

Up-to-now RNA interference leading to gene-specific silencing was described in different organisms - nematode, Drosophila, fungi, plants.

The known approach for changes of genetic properties of an organism, that is based on RNA interference, uses the antisense RNA (asRNA) that is complementary to the mRNA of the selected gene in antiparallel orientation and is synthesized *in vitro* and introduced into organism [A. Fire, S-Q. Xu, M.K. Montgomery, S.V. Kostas, S.E. Driver, C.C. Mello, Nature, 1998, vol. 391, p. 806].

The described approach is carried out as follows:
1. A gene with pathogenic activity is selected;
2. A DNA construct possessing a selected gene or its cDNA (a sequence corresponding to mRNA), i.e. natural DNA, in the opposite polarity under the control of selected promoter is prepared. This permits to perform transcription of non-coding strand of the gene. For the generation of the construct different vectors are used possessing DNA sequences for selection of transformants, for efficient expression of the turned-over gene and for correct "inscribing" of the construct into chromosomal domains;
3. asRNA is synthesized *in vitro* on the construct and introduced into organism by different methods ( electroporation, injections, *per os*).

An important problem of this approach for changing of genetic properties of an organism by RNA interference is the often occurrence of reversions of the constructs designed for asRNA synthesis by rearrangements leading to stop of asRNA transcription and start of transcription of the sequences corresponding to mRNA-strand. Thus, instead of inhibition of activity of the selected gene an increased transcription of the gene could occur. Start of transcription of the sequences corresponding to mRNA-strand can also happen if in the target site of insertion of the construct the host promoter sequences transcribing the sense strand are present. The probability of such events is rather high.

The highness of reversions is illustrated by demonstrative experiments on transgenic organisms. The constructs in these cases were introduced with the opposite aim - to increase the activity of a selected gene. However, reversions by spontaneous activation of transcription from the opposite strand resulted in complete inhibition of gene activity instead of activation of its expression, i.e. to gene-specific silencing by RNA interference mechanisms [M.K. Montgomery, A. Fire, Trends in Genetics, 1998, vol.14, p. 255; P. Sharp, Genes & Development, 1999, vol.13, p.139].

### Disclosure of the invention

The approach is suggested for changing of genetic properties of an organism by RNA interference leading to gene-specific silencing of a selected gene by RNA molecules, that are complementary in a parallel orientation (pcRNA) to mRNA of the selected gene; pcRNA are synthesized *in vivo* or *in vitro* on the artificial DNA sequence possessing symmetrical nucleotide ordering (mirror inversion) in respect to the nucleotide sequence of the gene.

### Brief description of the drawings

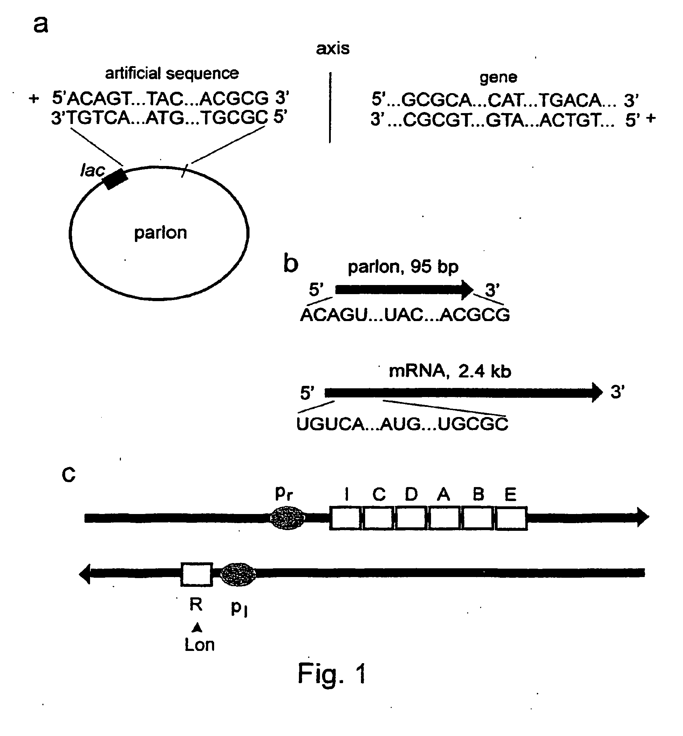

Fig. 1 shows the structure of the DNA conctruct possessing mirror nucleotide sequence in respect the *E. coli lon* gene and designed for expression of pcRNA, where:
   a - relationship between nucleotide sequence of the *lon* gene and the artificial chemically synthesized DNA possessing mirror nucleotide sequence in respect the gene (+ strands correspond to parlon pcRNA or mRNA); the synthesized DNA in the construct is under the control of the *lac* promoter driving the expression of parlon pcRNA;
   b - relationship between *lon* mRNA and parlon pcRNA;
   c - genes in the *lux*-regulon: pr and pl promoters of the *lux*-regulon are in different strands, genes are shown by the open bars; Lon protease cleaves the gene R product, that activates pr, and thus switches out the genes involved in the luminescence.

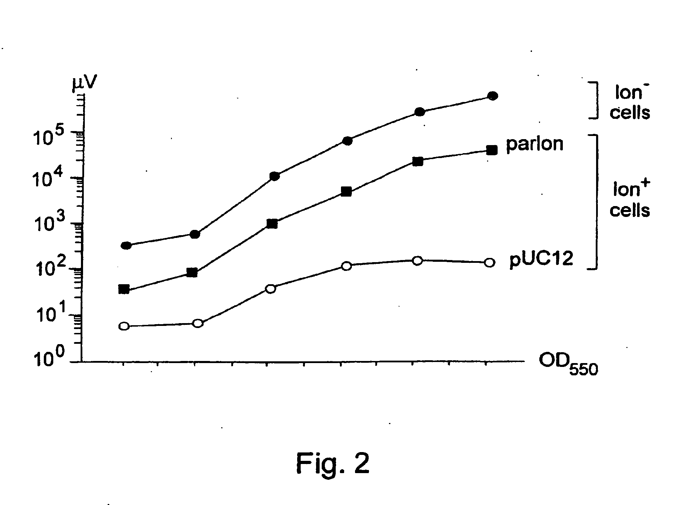

Fig. 2 shows the luminescence of *E. coli* cells that was observed after silencing of the *lon* gene induced by expression of parlon pcRNA, where:
   *lux*-regulon from *Vibrio fischeri* was introduced into *lon*⁺ or *lon⁻ E. coli* cells. In the resulted *lon*⁺ cells either the construct prepared in the pUC12 vector expressing parlon pcRNA or original pUC12 vectors without any insertion were introduced. The dependence of luminescence of the cells suspension presented as luminometer data in µV from optical density ( OD₅₅₀). Silencing of the *lon* gene induces the luminescence of the transformants expressing parlon construct.

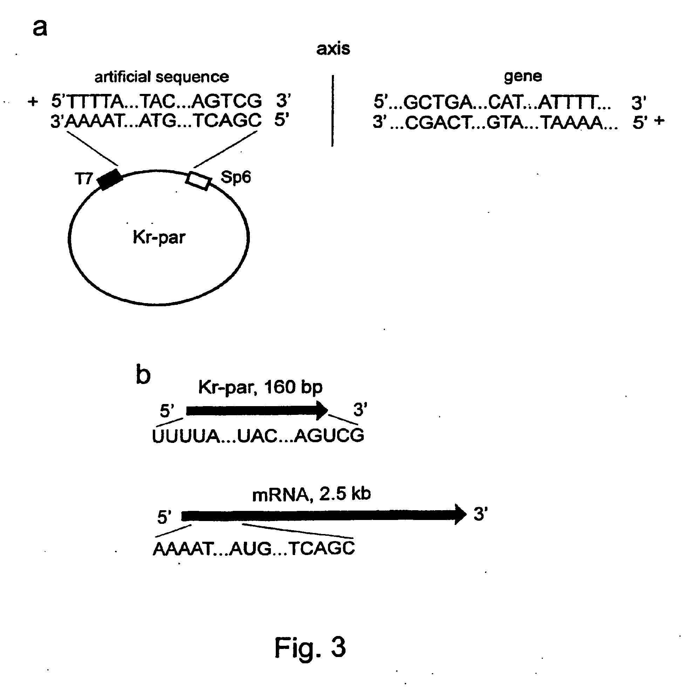

Fig. 3 shows the structure of the DNA conctruct possessing mirror nucleotide sequence in respect the *Drosophila Kruppel* gene and designed for expression of pcRNA (Kr-par), where:
   a - relationship between nucleotide sequence of the *Kruppel* gene and the artificial chemically synthesized DNA, possessing mirror nucleotide sequence in respect the gene (+ strands correspond to Kr-par pcRNA or mRNA); the synthesized DNA in the construct is under the control of the T7 RNA polymerase promoter driving the expression of Kr-par pcRNA (synthesis on the opposite strand of the construct is drived by SP6 RNA polymerase promoter);
   b - relationship between *Kruppel* mRNA and Kr-par pcRNA.

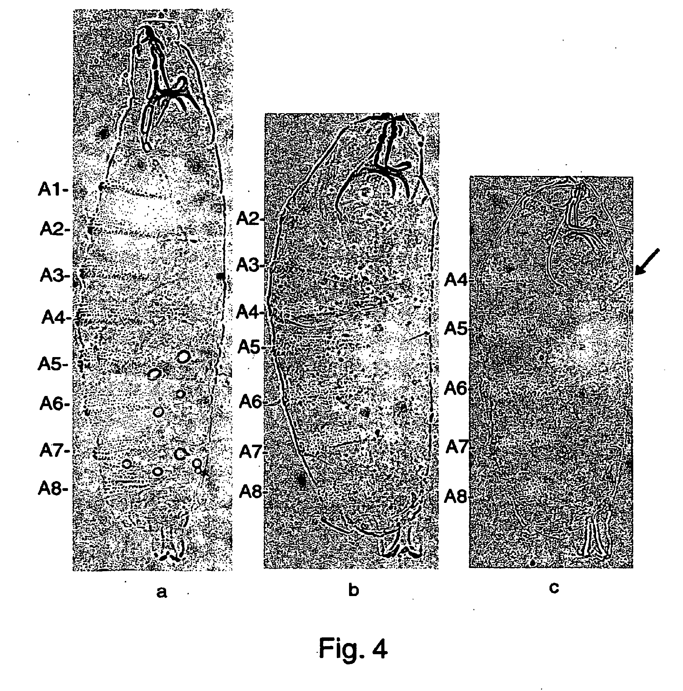

Fig. 4. shows the phenotypes of normal larva and *Kr* phenocopies generated after injections of the Kr-par pcRNA, where:
   a - phenotypes of normal *Drosophila* larva;
   b - phenotype of the larva developed after injection of the Kr-par pcRNA and possessing deletions of adjacent thoracic and the first abdominal segments;
   c - phenotype of the larva developed after injection of the Kr-par pcRNA and possessing deletions of adjacent thoracic and three anterior abdominal segments.
Arrow shows the atopic tracheal ending that is characteristic only for *Kr* phenotype.

### Best mode of carrying out the invention

The basis of the suggested invention was to increase the RNA interference reliability and to exclude the possibility of reversions leading to the synthesis of mRNA sequences on a construct designed for gene-specific silencing.

The approach is suggested for changing of genetic properties of an organism by RNA interference leading to gene-specific silencing of a selected gene by RNA molecules that are complementary in a parallel orientation (pcRNA) to mRNA of the selected gene; pcRNA are synthesized *in vivo* or *in vitro* on the artificial DNA sequence possessing symmetrical nucleotide ordering (mirror inversion) in respect to the nucleotide sequence of the gene.

This approach leads to more efficient RNA interference and gene-specific silencing and excludes the synthesis of mRNA sequences because in the constructs the non-homologous artificial DNA sequence is used.

The suggested approach is carried out as follows:
1. A gene with pathogenic activity (leading to a disease or interfering a buildup of biotechnological product) is selected;
2. Artificial DNA sequence is chemically synthesized, possessing the mirror nucleotide ordering in respect to the selected gene or its fragment;
3. A DNA construct is prepared on the basis of different vectors, possessing the chemically synthesized DNA sequence under the control of appropriate promoter, and a number of sequences important for efficient expression of the insert, and for correct "inscribing" of the construct in chromosomal domains;
4. The construct is introduced into organism by different methods for *in vivo* synthesis of pcRNA (transformation), or pcRNA is synthesized *in vitro* and used for injections.

The invention is clarified by the examples describing the realization of the suggested approach for changing of genetic properties of organisms (in which the changes of phenotype are observed) and illustrated by 4 figures.

### The data supporting the feasibility of the invention

The following examples are preferable and aimed only to confirm the feasibility of the invention and cannot be used as an argument for the restriction of the capacity of the Inventor's claims. Expert in the field will find easily another applications for the invention, which are undoubtedly covered by the Inventor's claims stated in the Claim listed below.

### Example 1. RNA interference in Escherichia coli induced by in vivo synthesized RNA molecules that are complementary in the same polarity to mRNA of the lon gene.

The *lon* gene was selected as far as it plays a key role in different regulatory events in *E. coli* cells.

Artificial DNA 95-bp (bp - base pair in DNA or RNA) sequence possessing the mirror nucleotide ordering in respect to the region of the *lon* gene was chemically synthesized.

This DNA was used for the pUC12-construct in which the strand expressing the parlon pcRNA is under the control of the *lac* promoter (Fig. 1 a, b).

The construct was introduced into *E. coli* cells by transformation.

The impact of parlon pcRNA expression on the activity of the endogenous *lon* gene was measured by effect of the latter on the activity of the *lux*-regulon that was introduced into *E. coli* cells from *Vibrio fischeri*. Lon protease is a negative regulator of the *lux*-regulon because it specifically cleaves the LuxR protein. The latter forms the complex with autoinductor and finally activates expression of the proteins involved in the luminescence. Promoters pr and pl of the *lux*-regulon are located in different strands, genes involved in the luminescence are shown by the open bars (Fig. 1 c). Actively expressing *lon* gene inhibits the transcription in the *lux*-regulon, that phenotypically is observed as inhibition of the luminescence. In contrast, silencing of the *lon* gene leads to increase of the LuxR concentration and to activation of the transcription in the *lux*-regulon and, consequently, to considerable enhancement of the luminescence of the cells. *Lux*-regulon, as 16 kb BamHI DNA fragment from *Vibrio fischer,i* was introduced into *lon*⁺ cells *E. coli* K12 AB1157 or *lon*⁺ cells *E. coli* K12 AB1899 (*lonl*).

The resulted *lon*⁺ cells are transformed either by the pUC12-construct expressing parlon pcRNA, or by pUC12 vector without any insert. Silencing of the *lon* gene is measured by enhancement of the luminescence of the cells. The luminescence of the cells expressing the parlon pcRNA is increased in several orders of the magnitude in comparison with the control pUC12-containing cells (Fig. 2).

The parlon pcRNA expressing transformants grown on plates reveal the characteristic property of the *lon*⁻ phenotype or for the silenced *lon* gene and form rather mucous colonies.

### Example 2. Generation of Kruppel (Kr) phenocopies by injections into Drosophila embryos of in vitro synthesized RNA, that is complementary in the same polarity to mRNA.

*Kr* is a homeotic gene, that is active in zygote and controls segment formation at the early embryonic stage of *Drosophila* development, was selected as a model allowing one to observe the early development in a multicellular organism. *Kr* mutants have deletions of the adjacent thoracic and anterior abdominal segments. Phenotypically this is observed just after cuticula formation and hatching of the larvae. *Kr* mutants have deletions of the adjacent thoracic and from one to several anterior abdominal segments and, sometimes, the development of actopic tracheal ending in the anterior part of the larva [E. Weischaus, C. Nusslein-Volhard, H. Kluding, Development, 1984, vol. 104, p. 172]. Thereafter, the *Kr* mutants have a unique phenotype developed as early as one day, that gives an advantage for study of the effect of RNA on a phenotype. Additional argument in favor of this model was that the study by antisense RNA injections was performed earlier [U.B. Rosenberg, A. Preiss, E. Seifert, H. Jackle, D.C. Knippe, Nature, 1985, vol. 313, p. 703].

Artificial DNA 160-bp sequence possessing the mirror nucleotide ordering in respect to the region of the *Kr* gene was chemically synthesized (Fig. 3 a, b). It should be stressed that antisense RNAs are synthesized on the non-coding strand of the same gene, while pcRNA can be synthesized only on the heterologous artificial DNA possessing the mirror order of nucleotide sequence.

The chemically synthesized artificial DNA is used for the construct in the pGEM-1 vector allowing to perform pcRNA synthesis with T7 RNA polymerase. pcRNA was denoted as Kr-par because it is complementary in a parallel orientation to *Kr* mRNA.

Embryos of the Oregon RC line are injected with pcRNA samples in the posterior pole at the syncytial stage and incubated under the water at 25 °C for 18-24 h. Then cuticula mounts are prepared and studied under the phase-contrast microscope.

The development of the larvae possessing typical *Kr* phenotype were observed. In control experiments, after injections of RNA synthesized on the opposite strand of the same construct with SP6 RNA polymerase, the development of normal larvae were observed.

Fig. 4 shows the normal larva (a) and two larvae developed after injections of Kr-par preparation (b, c). The latter have deletions of the thoracic and one or three abdominal segments and actopic tracheal ending in the anterior part of the larva, that is typical for *Kr* phenotype. The frequencies of observed phenocopies are about the same value as after injections with asRNA [U.B. Rosenberg, A. Preiss, E. Seifert, H. Jackle, D.C. Knippe, Nature, 1985, vol. 313, p. 703]. This demonstrats that pcRNA effects the expression of a key gene for differentiation in a multicellular organism, and a directed change of genetical properties of organism is attained.

Thus, the effect of nucleic acids possessing mirror inversions of nucleotide sequences and introduced by different ways in an organism on the phenotype is revealed. The main advantage of the suggested approach is that mirror sequences are capable of pcRNA synthesis and RNA interference but being heterologous cannot specify the corresponding mRNA sequence. That is why the utilization of this approach ("palindromic" approach), in contrast to regular approach using asRNA ("antisense" approach), cannot lead to reversion and to synthesis of the mRNA sequence.

### Industrial applicability

The invention suggested the general approach for changing of genetic properties of an organism and is based on the biological properties of mirror inversions of nucleotide sequences that are realized in RNA interference and gene-specific silencing. The suggested approach is based on the potent and specific biological activity of the transcripts coming from mirror inversions of nucleotide sequences in DNA, leads to changes in phenotype, and can be used in the gene-therapy in the medicine and the agriculture or in the industrial biotechnology for a gene-specific silencing of the disease-related genes or the genes interfering a buildup of a product, respectively.

## Claims

1. An RNA molecule that is complementary in a parallel orientation (pcRNA) to the mRNA of a selected gene, wherein said pcRNA is synthesized *in vivo* or *in vitro* from an artificial DNA sequence possessing symmetrical nucleotide ordering which is mirror inversion, in respect to the nucleotide sequence of the gene, for use as a medicament.

2. A DNA construct comprising an artificial DNA sequence encoding an RNA molecule that is complementary in a parallel orientation (pcRNA) to mRNA of a selected gene, wherein said pcRNA is synthesized *in vivo* or *in vitro* from said artificial DNA sequence possessing symmetrical nucleotide ordering which is mirror inversion, in respect to the nucleotide sequence of the gene, for use as a medicament.

3. A method for producing a DNA construct according to claim 2, comprising the steps of:
a) selecting a gene with a pathogenic activity;
b) chemically synthesizing an artificial DNA sequence possessing the mirror nucleotide ordering in respect to the selected gene or its fragment and;
c) preparing a vector comprising a DNA construct, which DNA construct possesses the chemically synthesized DNA sequence obtained in step b) under the control of an appropriate promoter, and sequences important for efficient expression of the insert and for correct inscribing of the construct in chromosomal domains.

4. A method for inhibiting or altering the expression of a gene in one or more *E.coli* cell(s) or Drosophila embryo comprising the steps of:
a) selecting a gene with a pathogenic activity;
b) chemically synthesizing an artificial DNA sequence possessing the mirror nucleotide ordering in respect to the selected gene or its fragment;
c) preparing a vector comprising a DNA construct, which DNA construct possesses the chemically synthesized DNA sequence under the control of an appropriate promoter, and sequences important for efficient expression of the insert and for correct inscribing of the construct in chromosomal domains, and;
d) introducing said construct obtained in step c) into said one or more *E.coli* cell(s) or Drosophila embryo for *in vivo* synthesis of pcRNA or transcribing pcRNA in vitro which is thereafter injected into said one or more *E.coli* cell(s) or Drosophila embryo.

## Patentansprüche

1. RNA-Molekül, das komplementär in einer parallelen Orientierung (pcRNA) zu der mRNA eines ausgewählten Gens ist, wobei die pcRNA *in vivo* oder *in vitro* aus einer künstlichen DNA-Sequenz synthetisiert wird, die symmetrische Nucleotidordnung besitzt, die bezüglich der Nucleotidsequenz des Gens Spiegelbild ist, zur Verwendung als Medikament.

2. DNA-Konstrukt, das eine künstliche DNA-Sequenz umfasst, die für ein RNA-Molekül codiert, das komplementär ist in einer parallelen Orientierung (pcRNA) zu mRNA eines ausgewählten Gens, wobei die pcRNA *in vivo* oder *in vitro* aus der künstlichen DNA-Sequenz synthetisiert wird, die symmetrische Nucleotidordnung besitzt, welche bezüglich der Nucleotidsequenz des Gens Spiegelbild ist, zur Verwendung als Medikament.

3. Verfahren zum Produzieren eines DNA-Konstrukts gemäß Anspruch 2, umfassend die Schritte:
a) Auswählen eines Gens mit einer pathogenen Aktivität;
b) chemisches Synthetisieren einer künstlichen DNA-Sequenz, die bezüglich des ausgewählten Gens oder seinem Fragment die Spiegelnucleotidordnung besitzt, und
c) Herstellen eines Vektors, umfassend ein DNA-Konstrukt, wobei das DNA-Konstrukt die chemisch synthetisierte DNA-Sequenz, die in Schritt b) erhalten wurde, unter der Kontrolle eines geeigneten Promotors besitzt, und Sequenzen, die für eine effiziente Expression des Inserts und für ein korrektes Inscribieren des Konstrukts in chromosomale Domänen wichtig sind.

4. Verfahren zur Inhibierung oder Veränderung der Expression eines Gens in einer oder mehreren *E. coli*-Zelle(n) oder in Drosophila-Embryo, umfassend die Schritte:
a) Auswählen eines Gens mit einer pathogenen Aktivität;
b) chemisches Synthetisieren einer künstlichen DNA-Sequenz, die die Spiegelnucleotidordnung bezüglich des ausgewählten Gens oder seines Fragments besitzt;
c) Herstellen eines Vektors, umfassend ein DNA-Konstrukt, wobei das DNA-Konstrukt die chemisch synthetisierte DNA-Sequenz unter der Kontrolle eines geeigneten Promotors besitzt, und Sequenzen, die für eine effiziente Expression des Inserts und für ein korrektes Inscribieren des Konstrukts in chromosomale Domänen wichtig sind, und
d) Einführen des in Schritt c) erhaltenen Konstrukts in eine oder mehrere *E. coli*-Zelle(n) oder Drosophila-Embryo zur in vivo-Synthese von pcRNA oder zum Transkribieren von pcRNA *in vitro*, die danach in die eine oder mehreren *E. coli*-Zelle(n) oder in Drosophila-Embryo injiziert wird.

## Revendications

1. Molécule d'ARN (ARNcp), qui est complémentaire, selon une orientation parallèle, de l'ARNm d'un gène sélectionné, ledit ARNcp étant synthétisé, *in vivo* ou *in vitro,* à partir d'une séquence d'ADN artificielle ayant un ordre de nucléotides symétrique, qui est une inversion en miroir de la séquences de nucléotides du gène, pour une utilisation comme médicament.

2. Construction d'ADN (ARNcp), comprenant une séquence d'ADN artificielle, codant pour une molécule d'ARN, qui est complémentaire, selon une orientation parallèle, de l'ARNm d'un gène sélectionné, ledit ARNcp étant synthétisé, *in vivo* ou *in vitro,* à partir d'une séquence d'ADN artificielle ayant un ordre de nucléotides symétrique, qui est une inversion en miroir de la séquences de nucléotides du gène, pour une utilisation comme médicament.

3. Procédé pour la production d'une construction d'ADN selon la revendication 2, comprenant les étapes consistant à :
a) sélectionner un gène ayant une activité pathogène ;
b) synthétiser, par voie chimique, une séquence d'ADN artificielle ayant l'ordre de nucléotides en miroir par rapport au gène sélectionné ou son fragment ; et
c) préparer un vecteur comprenant une construction d'ADN, cette construction d'ADN comprenant la séquence d'ADN synthétisée par voie chimique, obtenue dans l'étape b) sous le contrôle d'un promoteur approprié, et des séquences importantes pour une expression efficace du segment d'insertion, et pour une insertion correcte de la construction dans des domaines chromosomiques.

4. Procédé pour empêcher ou modifier l'expression d'un gène dans une ou plusieurs cellules de *E.coli* ou un embryon de drosophile, comprenant les étapes consistant à :
a) sélectionner un gène ayant une activité pathogène ;
b) synthétiser, par voie chimique, une séquence d'ADN artificielle ayant l'ordre de nucléotides en miroir par rapport au gène sélectionné ou son fragment ; et
c) préparer un vecteur comprenant une construction d'ADN, cette construction d'ADN comprenant la séquence d'ADN synthétisée par voie chimique, sous le contrôle d'un promoteur approprié, et des séquences importantes pour une expression efficace du segment d'insertion, et pour une insertion correcte de la construction dans des domaines chromosomiques ; et
d) introduire ladite construction obtenue dans l'étape c) dans lesdites une ou plusieurs cellules de *E. coli* ou l'embryon de drosophile, pour la synthèse, *in vivo,* d'un ARNcp, ou la transcription, *in vitro,* d'un ARNcp qui est ensuite injecté dans lesdites une ou plusieurs cellules de *E.coli* ou l'embryon de drosophile.
